# EUROPEAN PATENT APPLICATION

(11) **EP 2 022 403 A1**
(43) Date of publication of application: **11.02.2009**
(21) Application number: 08012966.1
(22) Date of filing: 17.07.2008
(51) Int. Cl.: A61B 6/08, A61B 6/00

(54) **Alignment apparatus for imaging system**

(30) Priority: 06.08.2007 US 834234; 13.03.2008 US 47608
(71) Applicant: Carestream Health, Inc., Rochester, NY 14608-1733 (US)
(72) Inventor: Wang, Xiaohui, New York 14534 (US); Rogers, Michael K., New York 14506 (US)
(74) Representative: Wagner, Karl H.

(57) **Abstract**

An apparatus for aligning a radiation source with an image receiver. The apparatus includes first and second light sources. The first light source is coupled to the image receiver and actuable to direct a first pattern of light toward the radiation source. The second light source is coupled to the image receiver and actuable to direct a second pattern of light toward the radiation source. The spatial relationship of the two patterns on or near the radiation source indicates a predetermined alignment position for the radiation source.

## Description

### FIELD OF THE INVENTION

This invention relates to apparatus for radiation imaging, having a positioning apparatus for providing proper centering alignment of the radiation source relative to an image detection device for recording a radiation image.

### BACKGROUND OF THE INVENTION

When an x-ray image is obtained, there is generally an optimal angle between the radiation source and the two dimensional receiver that records the image data. In most cases, it is preferred that the radiation source provides radiation in a direction that is perpendicular to the surface of the recording medium of the receiver. For this reason, large-scale radiography systems mount the radiation source and the recording medium holder at a specific angle relative to each other. Orienting the source and the receiver typically requires a mounting arm of substantial size, extending beyond the full distance between these two components. With such large-scale systems, unwanted tilt or skew of the receiver is thus prevented by the hardware of the imaging system itself.

With the advent of portable radiation imaging apparatus, such as those used in Intensive Care Unit (ICU) environments, a fixed angular relationship between the radiation source and two-dimensional radiation receiver is no longer imposed by the mounting hardware of the system itself. Instead, an operator is required to aim the radiation source toward the receiver surface, providing as perpendicular an orientation as possible, typically using a visual assessment. In computed radiography (CR) systems, the two-dimensional image-sensing device itself is a portable cassette that stores the readable imaging medium. In direct digital radiography (DR) systems, the two-dimensional image-sensing device is a digital detector with either flat, rigid, or flexible substrate support.

There have been a number of approaches to the problem of providing methods and tools to assist operator adjustment of source and receiver angle. One classic approach has been to provide mechanical alignment in a more compact fashion, such as that described in U.S. Patent No. 4,752,948 entitled "Mobile Radiography Alignment Device" to MacMahon. A platform is provided with a pivotable standard for maintaining alignment between an imaging cassette and radiation source. However, complex mechanical solutions of this type tend to reduce the overall flexibility and portability of these x-ray systems. Another type of approach, such as that proposed in U.S. Patent No. 6,422,750 entitled "Digital X-ray Imager Alignment Method" to Kwasnick et al. uses an initial low-exposure pulse for detecting the alignment grid; however, this method would not be suitable for portable imaging conditions where the receiver must be aligned after it is fitted behind the patient.

Other approaches project a light beam from the radiation source to the receiver in order to achieve alignment between the two. Examples of this approach include U.S. Patents No. 5,388,143 entitled "Alignment Method for Radiography and Radiography Apparatus Incorporating Same" and U.S. Patent No. 5,241,578 entitled "Optical Grid Alignment System for Portable Radiography and Portable Radiography Apparatus Incorporating Same", both to MacMahon. Similarly, U.S. Patent No. 6,154,522 entitled "Method, System and Apparatus for Aiming a Device Emitting Radiant Beam" to Cumings describes the use of a reflected laser beam for alignment of the radiation target. The methods presented use light to align the film or CR cassette or DR receiver. The '143 and '578 MacMahon disclosures describe a fixed Source-to-Image Distance (SID) be determined beforehand, then apply triangulation with this fixed SID value. Changing the SID requires a number of adjustments to the triangulation settings. This arrangement is not suited for portable imaging systems that allow a variable SID. Devices using lasers, such as that described in the '522 Cumings disclosure can require much more precision in making adjustments than is necessary.

Other examples in which light is projected from the radiation source onto the receiver are given in U.S. Patent No. 4,836,671 entitled "Locating Device" to Bautista and U.S. Patent No. 4,246,486 entitled "X-ray Photography Device" to Madsen. The Bautista '671 and Madsen '486 approaches use multiple light sources that are projected from the radiation source and intersect in various ways on the receiver.

One feature to the approaches described in MacMahon '143 and '578, Cumings '522, Bautista '671, and Madsen '486 is the need for some amount of redesign or retrofit of the radiation source itself. The light sources that direct light in various ways onto the cassette or receiver must be attached to the radiation source and then adjusted or calibrated in order to perform their alignment function. This requires mounting hardware, modification of existing equipment, and routing of power and control lines to the various light sources. This problem is a disadvantage. Moreover, even if this approach is used for a particular manufacturer's system, other radiology equipment cannot readily take advantage of the alignment apparatus that is used.

Today's portable radiation imaging devices allow flexibility for placement of the film cassette, CR cassette, or Digital Radiography (DR) receiver by the radiology technician. The patient need not be in a horizontal position for imaging, but may be at any angle, depending on the type of image that is needed and on the ability to move the patient for the x-ray examination. The technician can manually adjust the position of both the cassette and the radiation source independently for each imaging session. Thus, it can be appreciated that an alignment apparatus for obtaining the desired angle between the radiation source and the surface of the image sensing device be able to adapt to whatever orientation is suited for obtaining the image. Tilt sensing, as has been conventionally applied and as is used in the device described in U.S. Patent No. 7,156,553 entitled "Portable Radiation Imaging System and a Radiation Image Detection Device Equipped with an Angular Signal Output Means" to Tanaka et al. and elsewhere, does not provide sufficient information on cassette-to-radiation source orientation, except in the single case where the cassette is level. More complex position sensing devices can be used, but can be subject to sampling and accumulated rounding errors, requiring frequent resynchronization.

Thus, it is apparent that conventional alignment solutions may be workable for specific types of systems and environments; however, considerable room for improvement remains. Portable radiography apparatus are preferably compact and lightweight, which makes the mechanical alignment approach such as that given in the '948 MacMahon disclosure less desirable. The constraint to direct line-of-sight alignment reduces the applicability of many types of reflected light based methods to a limited range of imaging situations. The complex sensor and motion control interaction required by the Tanaka et al. '553 solution would add expense, complexity, weight, and size to existing designs, with limited benefits. Many less expensive portable radiation imaging units do not have the control logic and motion coordination components that are needed in order to achieve the necessary adjustment. These approaches do not give the operator the needed information for making a manual adjustment that is in the right direction for correcting misalignment.

Importantly, none of these conventional solutions described earlier is particularly suitable for retrofit to existing portable radiography systems. That is, implementing these configurations could be prohibitive in practice for all but newly manufactured equipment and could have significant cost impact.

Yet another issue not addressed by many of the above solutions relates to the actual working practices of radiologists and radiological technicians. A requirement for perpendicular delivery of radiation, given particular emphasis in the Tanaka et al. '553 application, is not optimal for all types of imaging. There are some types of diagnostic images for which an oblique (non-perpendicular) incident radiation angle is desired. For example, for the standard chest anterior-posterior (AP) view, the recommended central ray angle is oblique from the perpendicular (normal) by approximately 3-5 degrees. Conventional alignment systems, while they provide for normal incidence of the central ray, do not adapt to assist the technician for adjusting to an oblique angle.

Thus, it can be appreciated that there is a need for an apparatus that enables proper angular alignment of a radiation source relative to an image detection device for recording a radiation image.

### SUMMARY OF THE INVENTION

An object of at least one embodiment of the present invention is to provide an alignment apparatus that is suitable for a portable radiation imaging system.

With this object in mind, the present invention provides an apparatus for aligning a radiation source with an image receiver comprising: a first light source coupled to the image receiver and actuable to direct a first pattern of light toward the radiation source; and a second light source coupled to the image receiver and actuable to direct a second pattern of light toward the radiation source, wherein the spatial relationship of the two patterns on or near the radiation source indicates a predetermined alignment position for the radiation source. The spatial relationship indicative of desired alignment is determined by observing the proximity or the intersection of the two patterns.

A feature of the present invention is that it uses patterns of light directed toward the radiation source from the receiver.

An advantage of the present invention is that it allows straightforward retrofitting for existing x-ray apparatus.

A further advantage of the present invention is that it provides a method that can be adapted for use with a variable SID distance.

The present invention differs from existing limited tilt sensing approaches that have been used in a variety of earlier radiography systems. The apparatus and method of the present invention provide a solution to the problem of radiation source-to-receiver alignment that can be used with a number of x-ray imaging systems.

These and other objects, features, and advantages of the present invention will become apparent to those skilled in the art upon reading the following detailed description when taken in conjunction with the drawings wherein there is shown and described an illustrative embodiment of the invention.

### BRIEF DESCRIPTION OF THE DRAWINGS

While the specification concludes with claims particularly pointing out and distinctly claiming the subject matter of the present invention, it is believed that embodiments of the invention will be better understood from the following description when taken in conjunction with the accompanying drawings:
FIG. 1A is a schematic, perspective view showing the relative relationship of the patient being imaged to basic components of a diagnostic imaging apparatus.
FIG. 1B is a schematic, perspective view showing important dimensional relationships for imaging system setup.
FIG. 1C is a schematic, perspective view showing exemplary out-of-alignment positioning.
FIG. 2A is a schematic, perspective view showing the operation of one portion of an alignment apparatus in one embodiment.
FIG. 2B is a schematic, perspective view showing another portion of an alignment apparatus that works in conjunction with the portion shown in FIG. 2A.
FIG. 2C shows combined operation of the portions of the system shown in FIGS. 2A and 2B.
FIGS. 3A and 3B are plan views of the collimator of the imaging apparatus shown in FIGS. 2A-2C, showing respective, separate patterns of light directed to the collimator to produce a spatial relationship of intersection of the patterns to indicate alignment in one embodiment.
FIGS. 3C, 3D, 3E, 3F, 3G, 3H, and 3I are plan views of the collimator of the imaging apparatus shown in FIGS. 2A-2C, showing various spatial relationships of intersection of the two patterns of light of FIGS. 3A and 3B in different positions of the patterns of light directed to the collimator.
FIGS. 3J, 3K, 3L, 3M and 3N are schematic plan views of the spatial relationships of non-intersecting patterns of light whose proximity at the collimator indicates alignment.
FIGS. 3O, 3P, 3Q, 3R and 3S are schematic plan views of the spatial relationships of patterns of light whose intersection at the collimator indicates alignment.
FIG. 4A is a front elevation view of a housing for mounting the light sources used in one embodiment.
FIG. 4B is a top view, partially broken away, of the housing, taken along line 4B-4B of FIG. 4A.
FIG. 4C is a side view of the housing, taken along line 4C-4C of FIG. 4A.
FIG. 4D is a front elevation view of a housing for mounting the light sources having an attached anti-scatter grid, as used in another embodiment.
FIG. 4E a side view of the housing, taken along line 4E-4E of FIG. 4D.
FIG. 5 is a top view of the alignment apparatus according to one embodiment, showing the effect of calibration adjustment.

### DETAILED DESCRIPTION OF THE INVENTION

The following is a detailed description of the preferred embodiments of the invention, reference being made to the drawings in which the same reference numerals identify the same elements of structure in each of the several figures.

Figures shown and described herein are provided to illustrate principles of operation of embodiments of the present invention and are not drawn with intent to show actual size or scale. Some omission of conventional parts and exaggeration of detail have been used in order to emphasize important structural and positional relationships and principles of operation. The term "indicium" is singular; "indicia" is plural.

Reference is made to commonly assigned, copending U.S. Serial No. 11/862,579 filed September 27, 2007 by Wang et al., entitled EXPOSURE CENTERING APPARATUS FOR IMAGING SYSTEM, and to commonly assigned, copending U.S. Serial No. 11/862,617 filed September 17, 2007 by Wang et al., entitled ALIGNMENT APPARATUS FOR IMAGING SYSTEM USING REFLECTIVE ELEMENT. The disclosures of these applications are incorporated by reference into the present application.

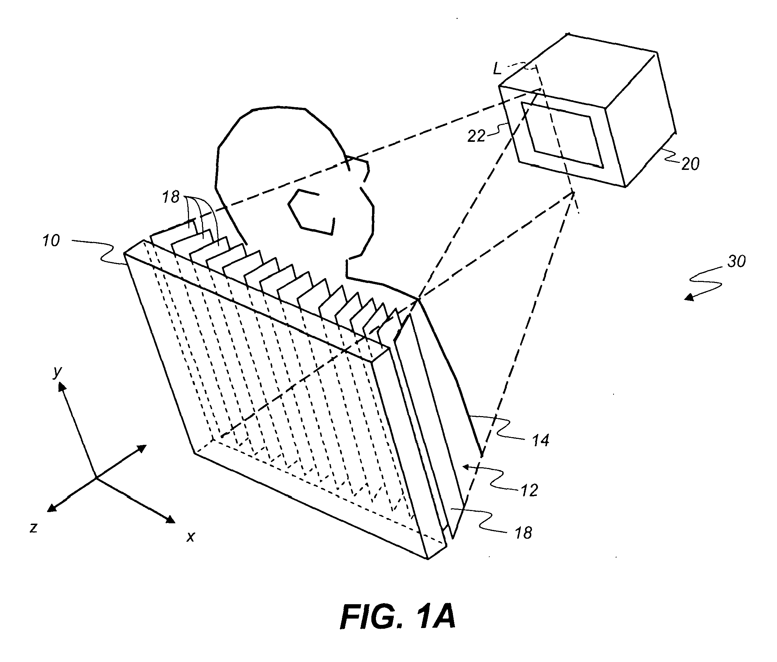

The perspective view of Figure 1A shows components of a diagnostic imaging apparatus 30. A radiation source 20, such as an x-ray source, directs radiation toward a patient 14. A receiver 10 positioned behind the patient forms the diagnostic image from the incident radiation passing through patient 14. Receiver 10 may have a photostimulable medium, such as a film or phosphor medium, for example, or may have a detector array that records an image according to radiation emitted from radiation source 20. An optional antiscatter grid 12 has essentially flat plates 18 arranged proximate to the surface of the receiver 10. Radiation source 20 has a collimator 22 that defines the radiation field that is directed toward receiver 10.

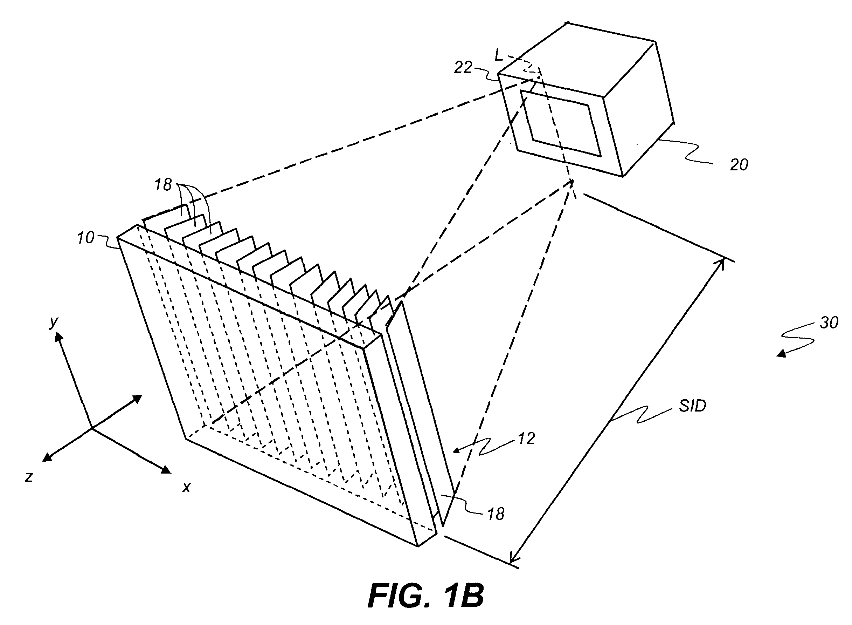

Radiation source 20 includes an adjustable angular orientation for directing radiation toward receiver 10. Figure 1B (with patient 14 not shown for visibility of system components) shows coordinate x-, y- and z-axes. Here, the source-to-image distance (SID) is in the general direction of the z axis. In Figure 1B, radiation source 20 is in its aligned position, at a suitable SID from receiver 10. Grid plates 18 are angularly arranged so that projections of their planes converge to define a focal line L at the SID. For best alignment in such an embodiment, radiation source 20 should be centered near focal line L and have the face portion of collimator 22 generally parallel to the planar surface of receiver 10.

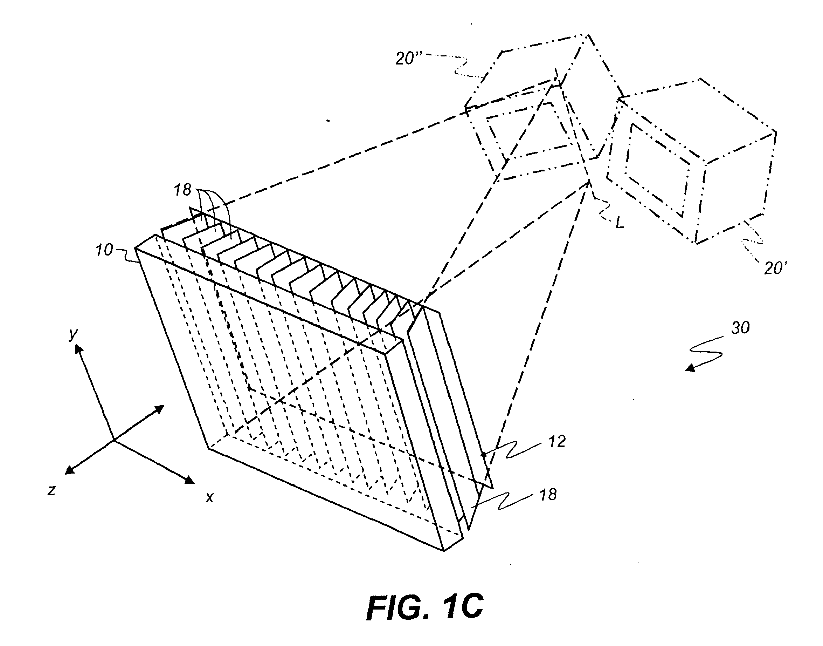

Figure 1C shows phantom outlines at 20' and 20" for poor positioning of radiation source 20. At positions 20' and 20", the SID is almost acceptable; however, radiation source 20 is not centered near focal line L and its angular orientation is badly skewed. Alignment of the radiation source with the grid would be poor at these and similar out-of-alignment positions, degrading image quality or, at worst, preventing a suitable diagnostic image from being obtained.

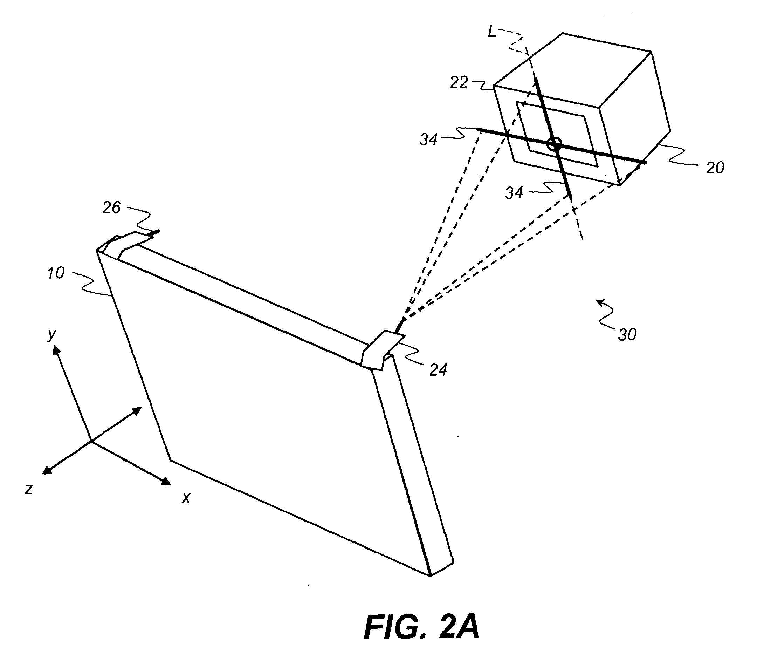

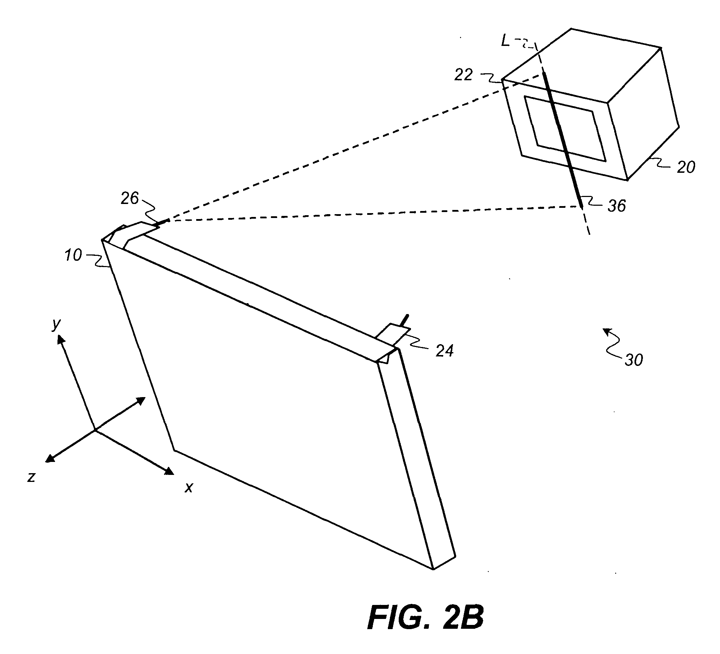

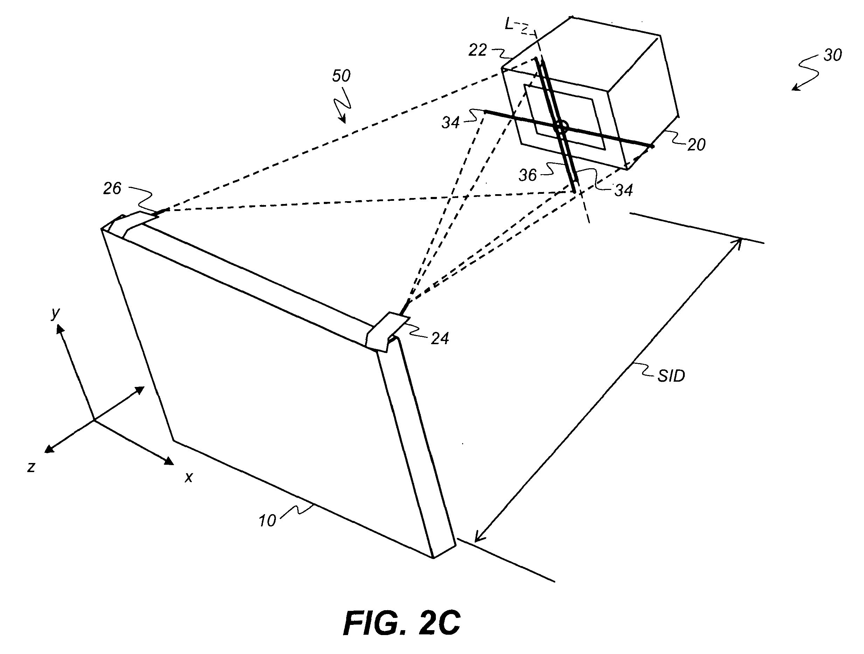

In Figures 2A, 2B, and 2C, grid 12 has been omitted for clarity, to show the two components of the system for which alignment is obtained according to embodiments of the present invention. A first light source 24, coupled to receiver 10, can be actuated, that is, energized, in order to direct a first pattern of light 34 toward collimator 22 of radiation source 20 in its correct position near focal line L, as shown in Figure 2A. Similarly, a second light source 26, also coupled to receiver 10, can be actuated/energized (turned on) to direct a second pattern of light 36 toward collimator 22 at focal line L. Figure 2C shows how patterns of light 34 and 36 intersect with a particular spatial relationship in an alignment apparatus 50 that uses two light sources 24 and 26 coupled to receiver 10. Alignment apparatus 50 formed by these devices provides alignment of receiver 10 and radiation source 20. When patterns of light 34 and 36 have a spatial relationship in which they intersect over at least some portion of space, the proximity of their intersection to focal line L is defined and the relative alignment of receiver 10 and radiation source 20 can be assessed.

In embodiments of the present invention, patterns 34 and 36 are directed so that they intersect on a surface that is on or near radiation source 20, as shown in Figure 2C. In the context of the present disclosure, the term "near" can mean within a radius distance of radiation source 20 that is less than about half the SID. In the particular embodiment shown in Figures 2A-2C, patterns 34 and 36 intersect on the surface of collimator 22 that lies along the front surface of radiation source 20. This particular embodiment takes advantage of the geometric relation between radiation source 20 and light sources 24 and 26 on receiver 10, where straightforward triangulation can be used. It can be appreciated that another surface in the near proximity of radiation source 20 could alternately be used for detection of patterns 34 and 36 and regarding their intersection. A suitable nearby surface could be provided, for example, by a handle that is used to manually adjust the position of radiation source 20. A grid layout or other visual target surface or other marking could alternately be provided, for example mounted on the side of or otherwise near radiation source 20 for detection of patterns 34 and 36.

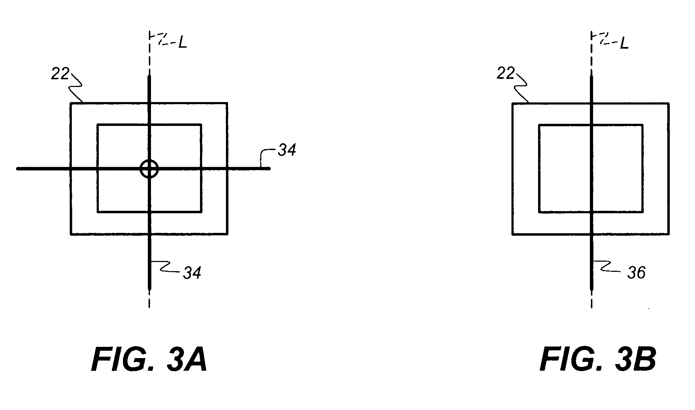

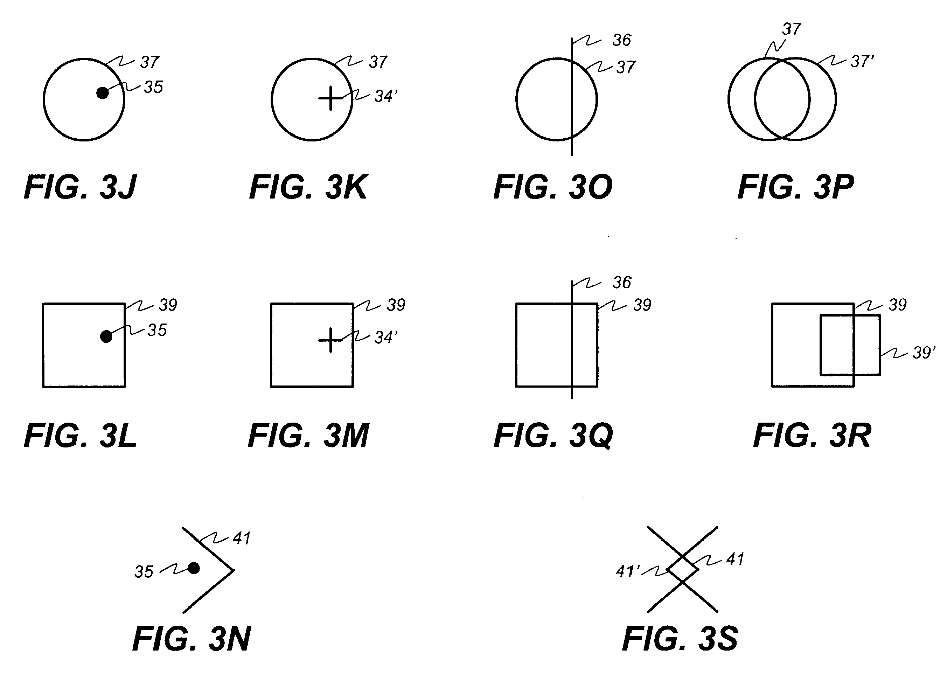

The plan views of Figures 3A through 3I show how patterns 34 and 36 cooperate to show relative alignment where they appear on the face of collimator 22 or other surface on or near radiation source 20 in one embodiment. In the embodiment shown, pattern 34 extends in two orthogonal dimensions and appears as a crosshairs pattern as it displays on the face portion of collimator 22, as shown in Figure 3A. Pattern 34 could alternately be a point of light (not extended in either height or width direction) or a line (extended in the line length direction ). Pattern 36, as shown in the particular embodiment of Figure 3B, is one-dimensional in that it extends in one dimension only (ignoring line width), but it could also be a point or a two-dimensional pattern. See the subsequent discussion of Figures 3J to 3S for other patterns of light suitable for use in the invention.

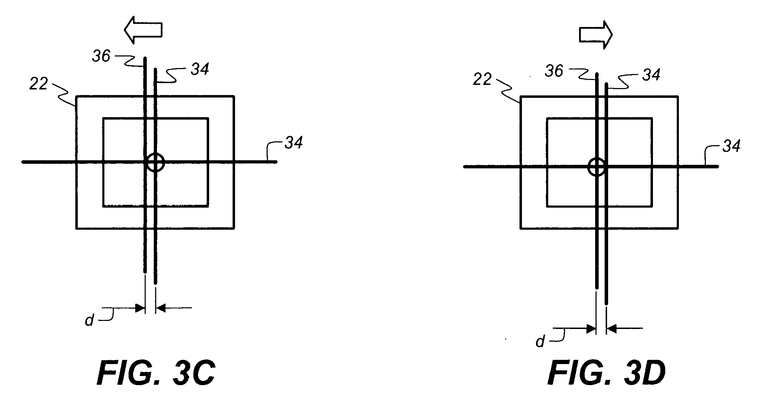

Figures 3C and 3D show how alignment of receiver 10 and radiation source 20 and any needed adjustment can be assessed using patterns 34 and 36 formed on the face of collimator 22. When substantially aligned, pattern 36 is essentially parallel with one of the crosshairs of pattern 34. However, an exacting level of alignment accuracy is seldom needed for x-ray imaging. In the examples of Figures 3C and 3D, there is some slight misalignment, indicated by the location of patterns 34 and 36 and their relative separation distance d. The pattern locations relative to the collimator center can indicate the need to adjust by moving radiation source 20 toward the left, as indicated by the block arrow in Figure 3C, or to the right, as indicated by the block arrow in Figure 3D. The magnitude of the relative distance d between patterns 34 and 36 indicates the need to move the radiation source 20 either closer to or further from receiver 10 in the z-direction, as distance d become smaller when the radiation source is closer to the focal line L.

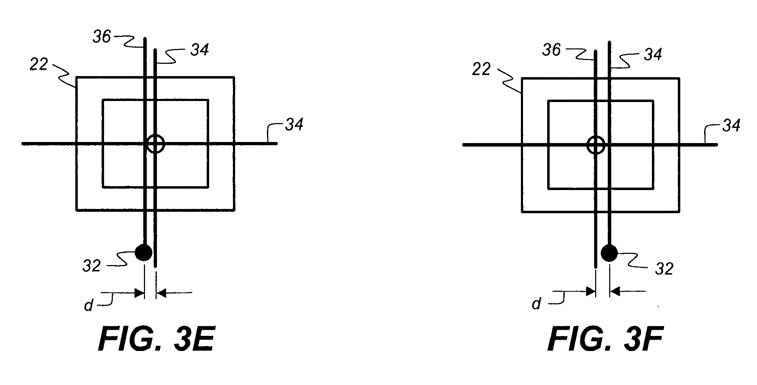

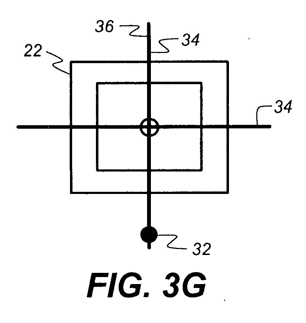

In some cases, the overlap of patterns 34 and 36 can be difficult to discern or detect; therefore, it may be difficult to know whether to move the radiation source closer or further in the z-direction. To assist, one or more indicia 32 can be provided as part of either or both patterns 34 and 36. Figures 3E, 3F, and 3G show one embodiment in which pattern 36 includes an indicium 32. In Figure 3E, light patterns 34 (projected from light source 24) and 36 (projected from light source 26) indicate that radiation source 20 needs to be moved further from the receiver 10 to cause the patterns to converge near the center of collimator 22. In Figure 3F, light patterns 34 and 36 indicate that radiation source 20 needs to move toward receiver 10 to cause convergence. In Figure 3G, the light patterns indicate that radiation source 20 and receiver 10 are optimally positioned with respect to each other.

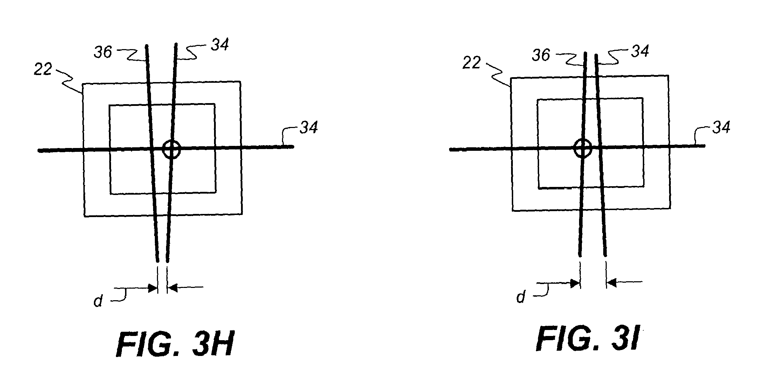

Figures 3H and 3I show a condition in which patterns 34 and 36 are not parallel, indicating that the surface of collimator 22 is not parallel to the x-y-plane. The relative varying distance d can indicate the need to adjust by tilting the radiation source 20 downward (needed with the orientation shown in Figure 3H) or upwards (with the orientation shown in Figure 3I).

As shown in Figures 2A-2C, the grid focal line L is parallel to the y-axis, which allows the radiation source 20 to move along the grid focal line L, up or down within a small range without appreciable grid cut-off effects. As such, the cross-hair laser pattern 34, extending in two directions, may be unnecessary for light source 24. Instead, a light source similar to source 26 can be used to create two near-parallel lines at the surface of collimator 22 for alignment patterns. Neither light source needs to be extended in either one or two dimensions, these can be simple two light sources creating dots of light on the collimator 22 or other suitable nearby surface. In summary, any combination of light dot, line, cross-hair, or other pattern can be employed.

Figures 3J to 3S show other types of patterns of light having spatial relationships suitable for use with light sources 24, 26. In the embodiments of Figures 3J to 3N, the spatial relationship of the patterns of light is such that the patterns need not intersect to show proper alignment of the radiation source and the receiver. In Figure 3J, a point pattern of light 35 falls within a circular pattern of light 37 upon alignment. In Figure 3K, a crosshair pattern of light 34' falls within a circular pattern of light 37. In Figure 3L, a point pattern 35 falls within a closed rectangular pattern of light 39. In Figure 3M, a crosshair pattern 37' falls within a rectangular pattern 39. Each of patterns 37 and 39 is shown as a closed perimeter pattern; however, one or more breaks in the pattern would be permissible. Figure 3N shows a point pattern 35 falling within an area partly surrounded by an open geometric pattern 41.

Figure 3O shows a one-dimensional pattern of light 36 that intersects with a circular pattern 37 upon alignment. Figure 3P shows two circular patterns 37, 37' intersecting upon alignment. Figure 3Q shows a one-dimensional pattern 36 that intersects with a rectangular pattern 39. Figure 3R shows two rectangular patterns 39, 39' intersecting upon alignment. Figure 3S shows two open geometric patterns 41, 41' intersecting at alignment. As before, one or more breaks in patterns 37 and 39 would be permissible.

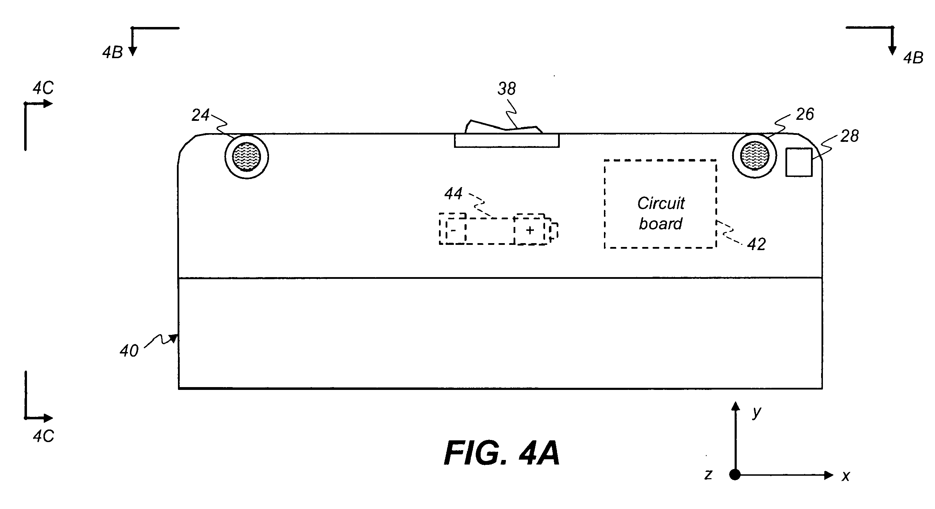

The apparatus for alignment apparatus 50 of the present invention is coupled to receiver 10 for forming patterns for alignment; it is not coupled to collimator 22. Figure 4A shows a front elevation view of a housing 40 for light sources 24 and 26 that form alignment apparatus 50 in one embodiment. Housing 40 can be fitted onto receiver 10 for use in setting up the imaging exam. Internal to housing 40 and shown in phantom are a circuit board 42 and a battery 44. Circuit board 42 can include a timer or other timing circuitry for controlling the actuation time of at least one of light sources 24 and 26 for an appropriate period or otherwise controlling the timing of light sources 24 and 26. Switch 38 can control or enable actuation of light sources 24 and 26. Battery 44 provides power for light sources 24 and 26. Battery 44 may be a rechargeable lithium ion cell in one embodiment.

Still referring to Figure 4A, an optional photosensor 28, such as a photodiode or similar component, can be provided for actuating light sources 24 and 26 when a collimator light signal or other light signal is sensed. With this arrangement, switch 38 is set to an "enable" position by the technician when receiver 10 is in place. In this enabled mode, photosensor 28 responds with a signal when it detects that the technician has turned on the collimator light. Logic circuitry on circuit board 42 then actuates light sources 24 and 26, possibly after a brief delay to allow time for the technician to move to the proper position for viewing patterns 34 and 36. Actuation can alternately be effected by a wireless signal, such as an RF or ultrasound signal. This wireless signal could be transmitted when the collimator light is turned on or at some other appropriate point in the x-ray setup workflow. Other sensing components could also be provided, such as sensors that prevent actuation of light sources 24 and 26 where the light path from these sources is obstructed.

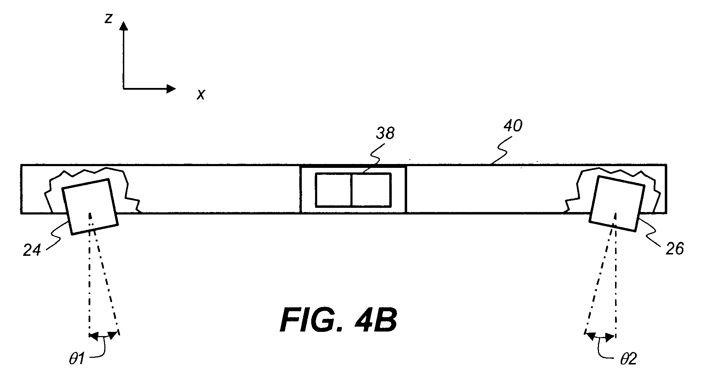

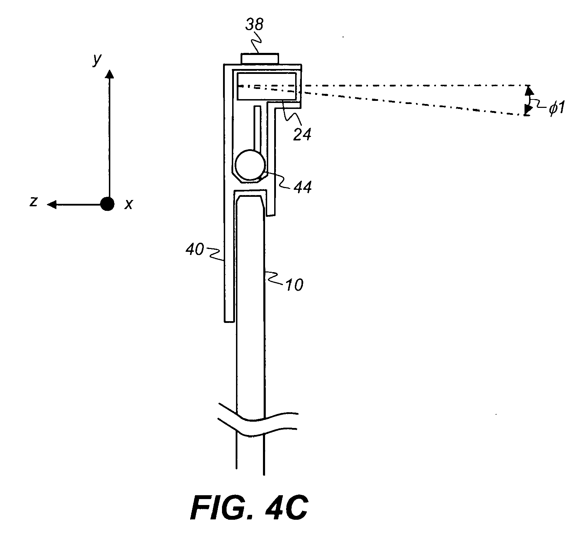

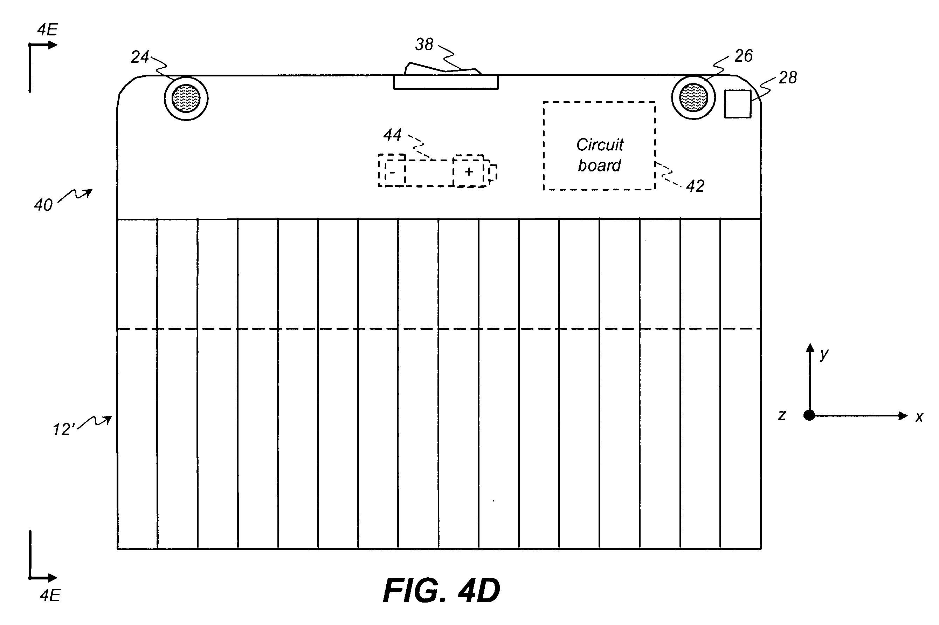

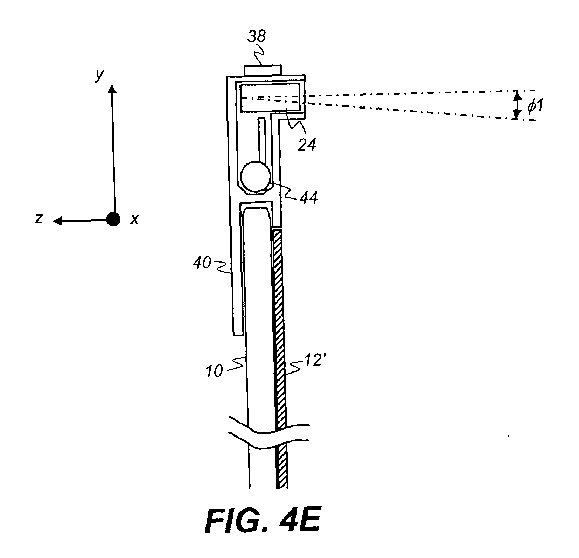

Figure 4B shows a top view of housing 40. As is shown, there is some horizontal angular displacement θ1 and θ2 of light sources 24 and 26 from normal (that is, from perpendicular to the front surface of housing 40 as shown in Figure 4A.) This angular offset directs the pattern of light from each source 24 and 26 to converge at the proper SID along focal line L, as was described earlier with reference to Figure 1B. As shown by angle φ1 in the side view of Figure 4C, there can be some vertical angular displacement of light sources 24, 26 from normal relative to the x-y plane, using the coordinate axes assignments shown. In the embodiment of Figure 4C, housing 40 fits onto the top of receiver 10. This arrangement has the advantage that, when properly seated, the patterns of light 34,36 will converge at focal line L due to the fixed angles of light sources 24 and 26. Any of a number of alternate embodiments are possible for coupling a detachable housing 40 to receiver 10, including embodiments using magnets or mechanical or hook-and-loop fasteners, for example. The use of housing 40 is advantaged because it provides the required spacing and angular orientation of the light sources; however, light sources 24 and 26 could be separately mounted on receiver 10 or could be built in to the receiver 10 panel. In an alternate embodiment shown in Figures 4D and 4E, light sources 24 and 26 are associated with a removable anti-scatter grid 12', to which the light sources are aligned. In one embodiment, grid 12' can be removed, shifted to the rear of receiver 10 when not in use. Light sources 24 and 26 can be re-oriented with the grid removed. In another embodiment, the light sources 24 and 26 are provided with on a fixture with a grid 12' built in, so when a grid is needed for x-ray exams the alignment apparatus 50 is always available with the grid.

Various light sources 24 and 26 can be employed. A third, fourth, or additional light source can be used for forming portions of patterns 34 and 36 or for forming additional patterns to aid alignment. In one embodiment, low-power laser diodes direct light through lenses in order to form patterns 34 and 36. Alternately, light-emitting diodes (LEDs) or other solid-state sources can be employed, as well as filament-based bulbs and other light sources. Light sources could be pulsed or continuous. One advantage of embodiments of the present invention relates to directing light from behind the patient toward collimator 22. Conventional solutions using light for alignment disadvantageously direct light from the collimator toward the patient and receiver panel. In addition, because light sources with conventional solutions are coupled to the radiation source or collimator housing, modification of the equipment is needed. The apparatus of the present invention is coupled to the receiver panel. This allows the apparatus of the present invention to be compatible with various radiography systems, without any modification to the system hardware.

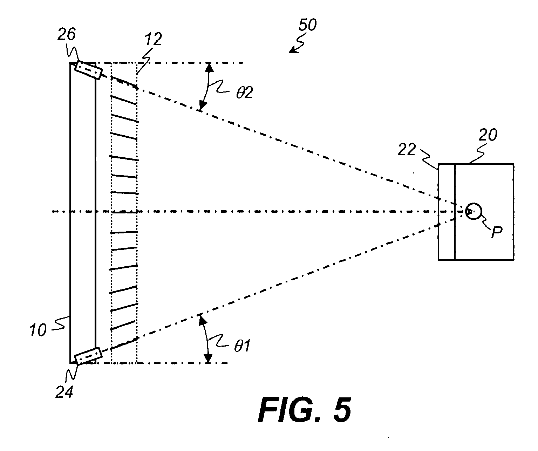

In practice, some initial calibration may be desirable to adapt alignment apparatus 50 to a particular radiography system. Referring to Figure 5, it is shown that the radiation is considered to be emitted from a point radiation source P that is not at the face of collimator 22, where the patterns from light sources 24 and 26 align, but is more precisely a point source within the body of the radiation source 20 housing. The actual distance between point source P and face portions of collimator 22 can differ from one system to the next. To compensate for system differences and for the relative location of the surface or surfaces where patterns 34 and 36 are formed, angles θ1 and θ2 can be adjusted.

Light sources 24 and 26 can be adjusted during a calibration procedure that aligns both patterns 34 and 36 at the proper SID and angular orientation for receiver 10. Light sources 24 and 26 could be designed to switch between two or more positions to be used with two or more different SIDs. These light sources could also be adjusted where a slight angular inclination is useful, such as for AP chest imaging, as described earlier.

It is instructive to describe the use of alignment apparatus 50 by an operator. The following sequence is given by way of example:
1. Position the patient with respect to receiver 10.
2. Mount housing 40 onto receiver 10.
3. Actuate light sources 24, 26 by setting switch 38 (Figures 4A-4C).
4. Move the radiation head into position so that patterns 34 and 36 (or patterns 34', 35, 36', 37, 37',39, 39' if in use) are in proper spatial relationship as projected onto the face of collimator 22 or onto some other suitable surface that is in near proximity to the radiation source.
5. Aim the collimator light onto the area of the patient that is to be imaged. Adjust collimator settings accordingly, using the collimator light as a guide. This step ensures that the radiation field is centered with respect to receiver 10 and that the field is left-right centered with light source 24 and 26.
6. Obtain the image.

In an alternate embodiment, step 3 may be used to enable light sources 24 and 26, but not to actuate them directly. Turning on the collimator light (step 5 in the sequence just given) would then actuate light sources 24 and 26. Then, the procedure given as step 4 for adjusting the position of the radiation head would be followed. It can be appreciated that there are a number of different alignment sequences that could be followed using the apparatus of the present invention.

The apparatus and method of the present invention allow the operator to visually ascertain the alignment of radiation source 20 with receiver 10 and to manually manipulate radiation source 20 into position until satisfactory alignment is achieved. Exact positioning is not a requirement for most diagnostic imaging environments; the operator can assess whether or not approximate alignment is close enough for obtaining the needed image. Automated methods, using motors or other actuators, sensors, and control logic, could be used for obtaining alignment in an alternative embodiment.

The invention has been described in detail with particular reference to certain preferred embodiments thereof, but it will be understood that variations and modifications can be effected within the scope of the invention as described above, and as noted in the appended claims, by a person of ordinary skill in the art without departing from the scope of the invention. For example, different methods could be used for coupling light sources 24 and 26 to receiver 10, temporarily or permanently, with or without the use of grid 12. Patterns 34 and 36 could have any number of arrangements for complementing each other to indicate alignment.

Thus, what is provided is an apparatus and method for providing proper centering alignment of the radiation source relative to an image detection device for recording a radiation image.

### PARTS LIST

10. Receiver
12, 12'. Anti-scatter grid
14. Patient
18. Plates
20, 20', 20". Radiation source
22. Collimator
24, 26. Light source
28. Photosensor
30. Diagnostic imaging apparatus
32. Indicium
34, 34'. Crosshairs pattern of light
35. Point pattern of light
36, 36'. One-dimensional pattern of light
37, 37'. Circular pattern of light
38. Switch
39, 39'. Rectangular pattern of light
40. Housing
41, 41'. Open geometric pattern of light
42. Circuit board
44. Battery
50. Alignment apparatus
d. Separation distance between 34, 36
L. Focal line
P. Point radiation source
Θ1, Θ2. Horizontal angles of displacement
Φ1. Vertical angle of displacement

### SUMMARY OF THE INVENTION

1. An apparatus for aligning a radiation source with an image receiver comprising:
   a first light source coupled to the image receiver and actuable to direct a first pattern of light toward the radiation source; and
   a second light source coupled to the image receiver and actuable to direct a second pattern of light toward the radiation source, wherein the spatial relationship of the two patterns on or near the radiation source indicates a predetermined alignment position for the radiation source.
2. The apparatus of 1 wherein the first pattern extends in two orthogonal dimensions.
3. The apparatus of 2 wherein the second pattern extends in a length direction.
4. The apparatus of 1 wherein the first pattern is a dot of light.
5. The apparatus of 4 wherein the second pattern of light is a closed perimeter pattern.
6. The apparatus of 4 wherein the second pattern of light is an open perimeter pattern.
7. The apparatus of 1 wherein the first and second patterns differ from each other.
8. The apparatus of 1 wherein the first and second light sources have a common housing that is coupled to the receiver.
9. The apparatus of 1 wherein at least one of the first and second light sources is a laser.
10. The apparatus of 1 wherein the first and second light sources are aligned with an antiscatter grid coupled to the receiver.
11. The apparatus of 10 wherein the grid defines the distance to a focal plane.
12. The apparatus of 1 wherein the radiation source is an x-ray source.
13. The apparatus of 1 wherein the first pattern of light is a cross-hair pattern.
14. The apparatus of 1 further comprising a timer for providing the first and second patterns of light for a predetermined duration.
15. The apparatus of 1 wherein the image receiver is a digital radiography panel.
16. The apparatus of 1 wherein at least one of the first and second light sources is pulsed.
17. The apparatus of 8 wherein the housing is coupled to an antiscatter grid.
18. The apparatus of 8 wherein the housing is magnetically coupled to the receiver.
19. The apparatus of 1 wherein the receiver is a cassette comprising a photostimulable medium.
20. The apparatus of 1 further comprising a timer for controlling the actuation time of at least the first light source.
21. The apparatus of 1 further comprising a photosensor for initiating actuation of the first and second light sources.
22. A method for aligning a radiation source with an image receiver comprising:
   directing a first pattern of light toward the radiation source from a first light source coupled to the image receiver;
   directing a second pattern of light toward the radiation source from a second light source coupled to the image receiver, wherein the spatial relationship of the two patterns on or near the radiation source indicates a predetermined alignment position for the radiation source relative to the image receiver; and
   adjusting the position of the radiation source according to the intersection of the two patterns.
23. The method of 22 wherein directing the first pattern of light comprises directing light from a laser.
24. The method of 22 wherein directing the first pattern of light comprises directing light from a solid-state light source.

## Claims

1. An apparatus for aligning a radiation source with an image receiver comprising:
a first light source coupled to the image receiver and actuable to direct a first pattern of light toward the radiation source; and
a second light source coupled to the image receiver and actuable to direct a second pattern of light toward the radiation source, wherein the spatial relationship of the two patterns on or near the radiation source indicates a predetermined alignment position for the radiation source.

2. The apparatus of claim 1 wherein the first pattern extends in two orthogonal dimensions.

3. The apparatus of claim 1 or 2 wherein the first pattern is a dot of light.

4. The apparatus of claim 3 wherein the second pattern of light is a closed or an open perimeter pattern.

5. The apparatus of any one of the preceding claims,wherein the first and second patterns differ from each other.

6. The apparatus of any one of the preceding claims,wherein the first and second light sources have a common housing that is coupled to the receiver.

7. The apparatus of any one of the preceding claims, wherein at least one of the first and second light sources is a laser.

8. The apparatus of any one of the preceding claims wherein the first and second light sources are aligned with an antiscatter grid coupled to the receiver.

9. The apparatus of any one of the preceding claims wherein the radiation source is an x-ray source.

10. The apparatus of any one of the preceding claims wherein the first pattern of light is a cross-hair pattern.

11. The apparatus of any one of the preceding claims further comprising a timer for providing the first and second patterns of light for a predetermined duration.

12. The apparatus of any one of the preceding claims wherein the receiver is a cassette comprising a photostimulable medium.

13. A method for aligning a radiation source with an image receiver comprising:
directing a first pattern of light toward the radiation source from a first light source coupled to the image receiver;
directing a second pattern of light toward the radiation source from a second light source coupled to the image receiver, wherein the spatial relationship of the two patterns on or near the radiation source indicates a predetermined alignment position for the radiation source relative to the image receiver; and
adjusting the position of the radiation source according to the intersection of the two patterns.

14. The method of claim 13 wherein directing the first pattern of light comprises directing light from a laser.

15. The method of claim 13 wherein directing the first pattern of light comprises directing light from a solid-state light source.
